# EUROPEAN PATENT APPLICATION

(11) **EP 4 000 651 A1**
(43) Date of publication of application: **25.05.2022**
(21) Application number: 21209806.5
(22) Date of filing: 23.11.2021
(51) Int. Cl.: A61L 2/10, B08B 3/00, B08B 5/00, B60S 3/00

(54) **DECONTAMINATION FACILITY FOR SHOPPING TROLLEYS**

(30) Priority: 24.11.2020 BE 202005848
(71) Applicant: BV D'heygere Philippe, 8930 Menen (BE)
(72) Inventor: STARÝ, Petr, CESKY TESIN (CZ); DUDA, Pavel, OSTRAVA (CZ)
(74) Representative: Hostens, Veerle

(57) **Abstract**

The present invention relates to a decontamination device (1) for decontaminating shopping trolleys (2), comprising:
- three decontaminating passages (3, 4, 5) which are arranged adjacent to each other and which are each provided with an access opening (13) through which at least one shopping trolley (2) to be decontaminated can be introduced into the decontaminating passage (3, 4, 5) for decontamination and through which the decontaminated contents of this decontaminating passage (3, 4, 5) can be removed from the decontaminating passage (3, 4, 5) after decontamination;
- a door (6) which is displaceable across the access openings (13) in order to alternately close off these access openings (13) in a closed state and open them up again in an open state;
- one or more decontaminating units (9) for alternately decontaminating the contents of each of the decontaminating passages (3, 4, 5) when the respective access opening (13) is in the closed state.

## Description

The present invention relates to a decontamination device for shopping trolleys.

Such a decontamination device is suitable for disinfecting one or more shopping trolleys, but may also be used to disinfect other objects, such as for example shopping baskets, luggage trolleys for airports, or service trolleys, wheelchairs, etc. Whenever shopping trolleys are discussed hereinafter, this also applies, *mutatis mutandis*, to any rolling object for which decontamination is desired.

Shopping trolleys are used consecutively by many different people to go shopping in stores.

Research also shows that many diseases can be transmitted via such shopping trolleys between people who handle these rolling objects, as a result of transmission of viruses and/or bacteria and/or other pathogens that can transmit infections from person to person.

In order to prevent this, it is important to disinfect these shopping trolleys between consecutive uses by various people in order to remove these viruses and/or bacteria and/or pathogens from the shopping trolleys.

Shopping trolleys are therefore typically disinfected between uses by users with the aid of disinfectants and paper towels. However, this is quite laborious. This is often ineffective too, given that, on the one hand, the effectiveness is dependent on the way the user disinfects the shopping trolley and, on the other hand, the disinfectants used are not always effective. In addition, many of the disinfectants used can result in increased wear of the shopping trolleys. Furthermore, many of the disinfectants used are harmful to the hands of the people using them to disinfect the shopping trolleys, and may lead to these people having allergic reactions.

A number of decontamination devices for automatically disinfecting shopping trolleys are also already known.

Several of these decontamination devices comprise:
- a decontaminating passage which is provided with an access opening through which at least one shopping trolley to be decontaminated can be introduced into the decontaminating passage, as contents for this decontaminating passage, for decontamination and through which the decontaminated contents of this decontaminating passage can be removed from the decontaminating passage after decontamination;
- a door which is displaceable between a position in which this door closes off the access opening in a closed state and a position in which this door opens up the access opening in an open state; and
- one or more decontaminating units for decontaminating the contents of the decontaminating passage when the access opening is in the closed state.

The present invention relates to such a decontamination device. EP 2 724 791 A1 describes and illustrates such a decontamination device, which comprises two of said decontaminating passages and has an alternating decontamination function.

In practice, such decontamination devices are not yet in widespread use. These decontamination devices need to be made more user-friendly.

This aim is achieved by providing a decontamination device for decontaminating shopping trolleys, comprising:
- a decontaminating passage which is provided with an access opening through which at least one shopping trolley to be decontaminated can be introduced into the decontaminating passage, as contents for this decontaminating passage, for decontamination and through which the decontaminated contents of this decontaminating passage can be removed from the decontaminating passage after decontamination;
- a door which is displaceable between a position in which this door closes off the access opening in a closed state and a position in which this door opens up the access opening in an open state;
- one or more decontaminating units for decontaminating the contents of the decontaminating passage when the access opening is in the closed state;
wherein said decontaminating passage is a first decontaminating passage of the decontamination device, wherein the decontamination device comprises two additional decontaminating passages which are arranged adjacent to the first decontaminating passage and which are each provided with an access opening through which at least one shopping trolley to be decontaminated can be introduced into this decontaminating passage, as contents for this decontaminating passage, for decontamination and through which the decontaminated contents of this decontaminating passage can be removed from the decontaminating passage after decontamination, wherein the access openings of the decontaminating passages are arranged adjacent to each other, wherein the door is displaceable across these access openings in order to alternately close off these access openings in a closed state and open them up again or leave them open in an open state, and wherein the one or more decontaminating units are provided for alternately decontaminating the contents of each of these decontaminating passages when the respective access opening is in the closed state.

The decontamination device further comprises a roof which is displaceable across the decontaminating passages in order to alternately close off each of the decontaminating passages at the top. In this case, the one or more decontaminating units are integrated into the displaceable roof.

The length of the decontaminating passages is preferably chosen as a function of the number of shopping trolleys to be decontaminated per unit time.

Such a decontamination device may be provided so as to in each case decontaminate one shopping trolley at a time in one of the decontaminating passages, while the other decontaminating passages are provided to respectively remove an already decontaminated shopping trolley and introduce a shopping trolley to be decontaminated. It is also possible for the decontaminating passages to be provided so as to have a plurality of shopping trolleys placed therein so that a plurality of shopping trolleys can be decontaminated at the same time.

The width of the decontaminating passages is preferably chosen as a function of the width of the shopping trolleys to be decontaminated.

The one or more decontaminating units may be provided in the form of an ozone generator, in order to decontaminate the shopping trolleys using ozone, or may be provided so as to decontaminate the contents of a decontaminating passage with the aid of another decontaminating gas and/or using atomized products and/or using decontaminating agents and/or using disinfectants and/or using UV light, etc.

The duration for which decontamination is performed can be chosen as a function of the volume of each decontaminating passage. This may take minutes, for example. By operating three separate decontaminating passages which are each used alternately for a subsequent step in the decontamination process, decontamination can be carried out in a quicker and safer manner than in the case of the already known solutions.

A displaceable door can be used to close off each decontaminating passage in order to decontaminate products that have been introduced therein. Thus, undesired entry into this space is not possible during the decontamination. Decontaminating agents can thus also be kept inside this space in an optimal manner so as to decontaminate the products to be decontaminated in an optimum manner.

Said door may be provided so as to be displaceable in various ways. Said door may be provided, for example, so as to be rollable and/or moveable and/or slidable, etc. This door may be produced in one-part form or may be divided into several parts and in this case, for example, into several segments.

In a preferred embodiment, the displaceable door is slidable across the access openings in order to alternately close off these access openings.

By providing a displaceable roof, it is also possible for the height of the decontaminating passages to be chosen as a function of the dimensions of the shopping trolleys to be decontaminated. This height can thus be adapted to the products to be decontaminated, and no longer has to be chosen as a function of the height of individuals who introduce these products into, or remove them from, the decontaminating passages. The space where the products are decontaminated can thus be optimally dimensioned as a function of the decontamination of these products.

Furthermore, one or more decontaminating units may also be integrated into the displaceable door.

The displaceable roof and the displaceable door preferably form part of the same displaceable roof/door unit. More specifically, such a roof/door unit may be manufactured with a folded metal sheet.

In a specific embodiment, the decontamination device comprises signalling means in order to indicate, for each decontaminating passage, whether:
- the access opening thereof is being closed, and when it is in the closed state, whether the contents of this decontaminating passage are being decontaminated; or
- the access opening thereof is open in order to remove the decontaminated contents of this decontaminating passage from the decontaminating passage through said access opening after decontamination; or
- the access opening thereof remains open in order to introduce at least one shopping trolley to be decontaminated through said access opening into the decontaminating passage, as contents for this decontaminating passage, after the decontaminated contents of this decontaminating passage have been removed from decontaminating passage through said access opening.

By way of example, such signalling means may comprise signalling signs and/or signalling lights, such as for example LED lights, and/or signalling sounds, etc.

In a particularly preferred embodiment, the decontamination device comprises a control unit for controlling the displacement movement of the displaceable door and for controlling the one or more decontaminating units in order to decontaminate the contents of each of the decontaminating passages.

In embodiments with said signalling means, such a control unit is preferably additionally provided for controlling the signalling means.

Such a control unit is preferably configured to control the displacement movement and the decontaminating units in such a way that the following alternately takes place in each case:
- the access opening of one of the decontaminating passages is closed off, and when it is in the closed state, the contents of this decontaminating passage are decontaminated;
- the access opening of one of the decontaminating passages is opened in order to remove the decontaminated contents of this decontaminating passage from the decontaminating passage through said access opening after decontamination; and
- the access opening of one of the decontaminating passages remains open in order to introduce at least one shopping trolley to be decontaminated through said access opening into the decontaminating passage, as contents for this decontaminating passage, after the decontaminated contents of this decontaminating passage have been removed from the decontaminating passage through said access opening.

During the first use of the day, it may be the case that all of the shopping trolleys are ready for use, even the shopping trolleys that are still in the closed decontaminating passage. All of the decontaminating passages are then preferably released sequentially, with it for example being possible to start with the decontaminating passage next to the closed decontaminating passage first.

Furthermore, a safety system may also be provided whereby it is not possible for any contaminated products to be taken into a passage with the products that have just been decontaminated.

It is moreover possible for an additional closure system to be provided, for example, in order to close off a decontaminating passage once a sufficient number of objects have been placed in this decontaminating passage so that it cannot be blocked as a result of too many objects.

Furthermore, it is preferably provided that an alarm is triggered when an access opening becomes blocked. The blockage may be a result of too many objects being placed in a decontaminating passage. This would mean the displaceable door can no longer be displaced.

The aim of the present invention is furthermore also achieved by providing a computer program product that consists of computer-readable code and that, when this code is executed by the control unit of an above-described decontamination device according to the present invention, has the effect that the latter is configured as described above. Moreover, the aim of the invention is achieved by providing a non-volatile, machine-readable storage medium in which such a computer program product is stored.

The present invention will now be explained in greater detail by means of the following detailed description of a preferred embodiment of a decontamination device according to the invention. The sole aim of this description is to give an illustrative example and to indicate further advantages and features of the present invention and can therefore by no means be interpreted as limiting the scope of application of the invention or of the patent rights defined in the claims.

In this detailed description, reference numerals are used to refer to the attached drawings, in which:

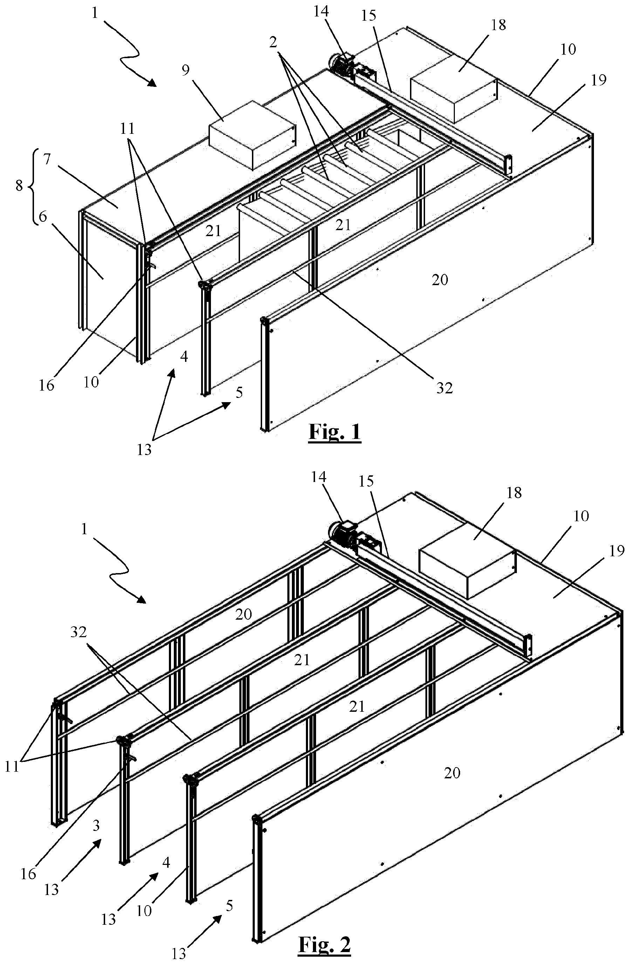

- Figure 1 illustrates an embodiment of a decontamination device according to the present invention in perspective;
- Figure 2 illustrates the decontamination device from Figure 1 in perspective, without the roof/door unit thereof;

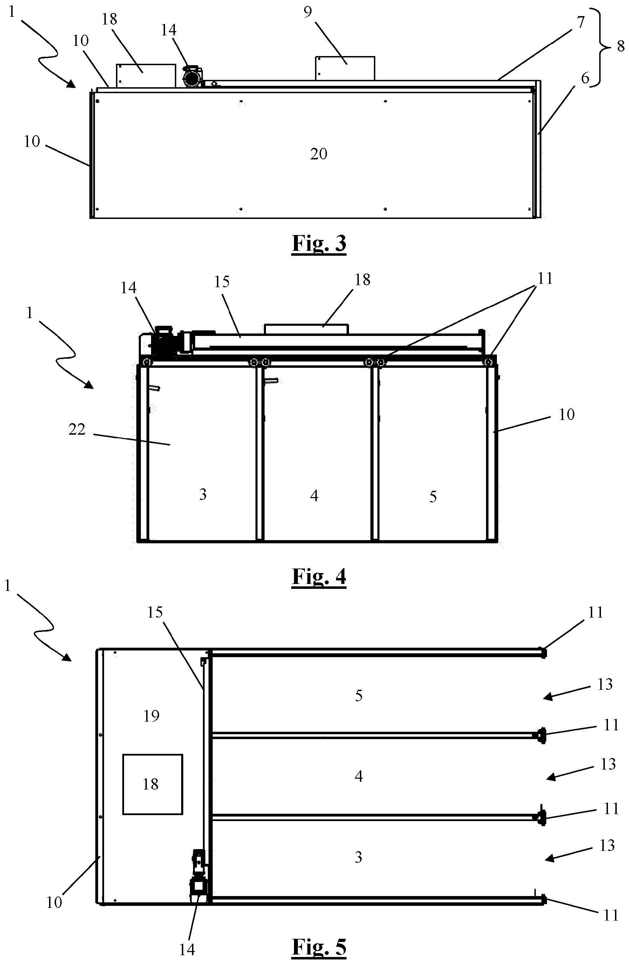

- Figure 3 shows the decontamination device from Figure 1 in side view;
- Figure 4 shows the decontamination device from Figure 1 in front view, without the roof/door unit thereof;
- Figure 5 shows the decontamination device from Figure 1 in top view, without the roof/door unit thereof;

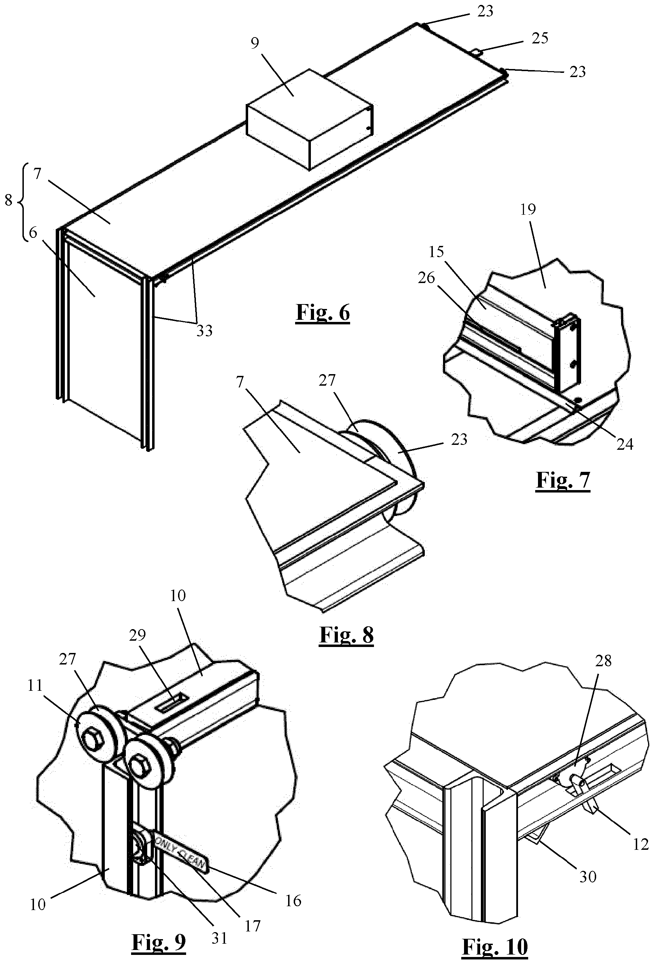

- Figure 6 shows a separate perspective view of the roof/door unit of the decontamination device from Figure 1;
- Figure 7 shows a more detailed perspective view of a part of the decontamination device from Figure 1 at the location of the connection of the third decontaminating passage to the fixed roof;
- Figure 8 shows a more detailed perspective view of a part of the roof/door unit of the decontamination device from Figure 1 at the location of a corner of the roof, which is provided for connection to the fixed roof;
- Figure 9 shows a more detailed perspective view of a part of the decontamination device from Figure 1 at the location of a front upper corner of the frame at the location of a partition wall;
- Figure 10 shows a more detailed perspective view of a part of the roof/door unit of the decontamination device from Figure 1 at the location of the connection of the door to the roof;

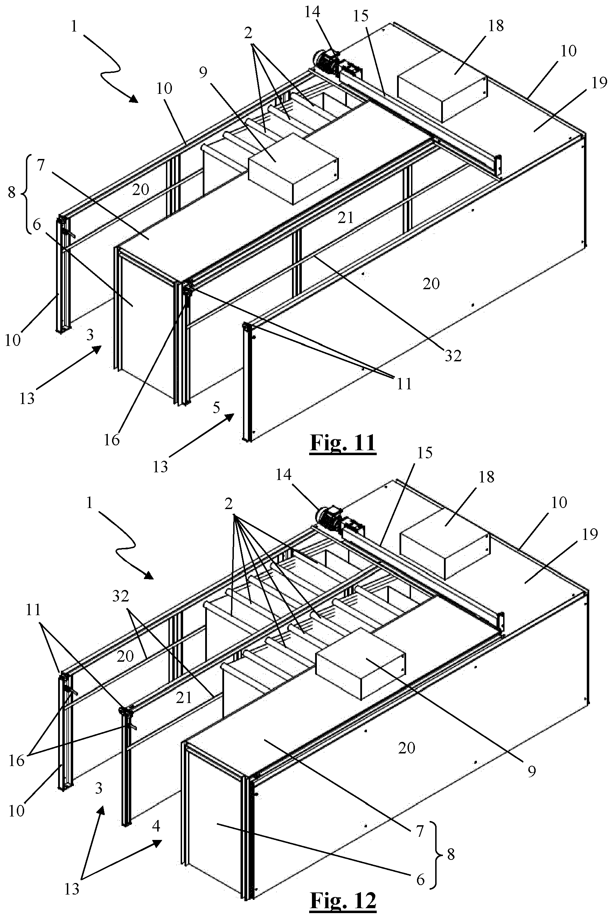

- Figure 11 illustrates the decontamination device from Figure 1 in perspective, the roof/door unit having been displaced across one access opening in relation to Figure 1;
- Figure 12 illustrates the decontamination device from Figure 1 in perspective, the roof/door unit having been displaced across two access openings in relation to Figure 1.

The illustrated decontamination device (1) is constructed from a frame (10) composed of metal profiles, to which two outer side walls (20) and two inner walls (21) are fastened. These walls (20, 21) may each be composed of one or more panels. Furthermore, a rear wall (22) and a fixed roof part (19) are fastened to this frame (10).

In this case, said walls (20, 21, 22) define three decontaminating passages (3, 4, 5). There is an access opening (13) to these decontaminating passages (3, 4, 5) on the front side of these decontaminating passages (3, 4, 5). A part of the top side of the decontaminating passages (3, 4, 5) is also open.

A displaceable roof/door unit (8) is provided which is slidable across the access openings (13) in order to alternately close off each of the decontaminating passages (3, 4, 5). This roof/door unit (8) is composed of a second frame (33) to which sheets are fastened. The door part (6) of the roof/door unit (8) is provided to close off the decontaminating passages (3, 4, 5) on the front side, while the roof part (7) of the roof/door unit (8) is provided to close off the decontaminating passages (3, 4, 5) on the rear side.

In order to slide the roof/door unit (8), a drive motor (14) is provided which drives a chain in a housing (15) via transmission means. The roof/door unit (8) is connected to this chain by means of an engagement pin (25) which extends through a slot (26) in the housing (15), in such a way that this chain entrains the roof/door unit (8) when it moves.

During the displacement movement, the roof/door unit (8) is guided with the aid of, on the one hand, guide wheels (11) which are rotatably mounted on the frame (10) and, on the other hand, guide wheels (23) which are rotatably mounted on the roof part (7). The guide wheels (11) are provided with a slot (27) on their circumference. The bottom of the roof part (7) of the roof/door unit (8) is provided with a guide profile (30) that engages with the wheels (11) on the frame (10) in order to guide the roof/door unit (8) on top of these wheels (11). The top of the fixed roof part (19) is provided with a guide rail (24) with which the wheels (23) of the roof part (7) engage in order for these wheels (23), and thus also the roof/door unit (8), to be guided thereon.

On top of the roof part (7), an ozone generator (9) is fitted in a housing in order to introduce ozone into a decontaminating passage (3, 4, 5) that has been closed off by means of the roof/door unit (8) in order to decontaminate shopping trolleys (2) placed therein. Here, instead of the ozone generator (9), it would for example also be possible for an atomizer to be provided in order to atomize decontaminating agents. It would furthermore alternatively be possible for UV lamps to be provided for this purpose, such as for example LED lamps, which are provided in adequate numbers to prevent shadows from occurring.

A central control unit (18) is provided on the fixed roof part (19) to control the decontamination device (1).

In each case one of the decontaminating passages (3, 4, 5) is selected in order to have the contents thereof decontaminated. To this end, the central control unit (18) then actuates the roof/door unit (8) to close off this decontaminating passage (3, 4, 5). When the roof/door unit (8) is positioned correctly, the central control unit (18) actuates a blocking element (12) at the bottom of the roof/door unit (8) with the aid of a motor (28) in order to make said blocking element engage with a corresponding slot (29) in the frame (10) so as to retain the roof/door unit (8) in this position.

After this decontaminating passage (3, 4, 5) has been closed off with the aid of the roof/door unit (8), the central control unit (18) actuates the ozone generator (9) to decontaminate the contents of the decontaminating passage (3, 4, 5). While the decontamination is being performed, the central control unit (18) actuates LED lighting to output a desired accompanying signal.

A second decontaminating passage (3, 4, 5) is in each case provided for removing the decontaminated shopping trolleys (2) from said decontaminating passage through the access opening (13) thereof after decontamination.

Both sides of the various decontaminating passages (3, 4, 5) are provided with guide rails (32) so that the shopping trolleys (2) can be brought smoothly into and out of the decontaminating passages (3, 4, 5) without damaging the walls (20, 21).

In order to release these decontaminated shopping trolleys (2), the central control unit slides the roof/door unit (8) from this second decontaminating passage (3, 4, 5) to the first decontaminating passage (3, 4, 5) as described above. To this end, the blocking element (12) is first released from the corresponding slot (29) in the frame (10) by using the central control unit (18) to actuate the motor (28). When this second decontaminating passage (3, 4, 5) is being released, the central control unit (18) actuates the motor (31) of the signalling sign (16) to place this signalling sign (16) in the accompanying position. LED lighting is also actuated to indicate a desired accompanying signal.

The third decontaminating passage (3, 4, 5) is in each case provided so as to refill this third decontaminating passage (3, 4, 5) with shopping trolleys (2) to be decontaminated after the decontaminated shopping trolleys (2) have been removed from it. The central control unit (18) ensures that this decontaminating passage (3, 4, 5) remains open for this purpose, actuates the motor (31) of the signalling sign (16) to place the latter in the desired accompanying position and also actuates LED lighting such that the latter outputs a desired accompanying signal.

The decontamination device (1) is provided with detection means to detect whether enough shopping trolleys (2) have been introduced into this third decontaminating passage (3, 4, 5). When enough shopping trolleys (2) are present, the central control unit (18) will actuate a closure system to close off this decontaminating passage (3, 4, 5) so that it cannot be blocked as a result of too many shopping trolleys (2). If too many shopping trolleys (2) are nevertheless introduced or if an object blocks the access opening (13), the central control unit (18) will trigger an alarm.

## Claims

1. Decontamination device (1) for decontaminating shopping trolleys (2), comprising:
- a first decontaminating passage (3) which is provided with an access opening (13) through which at least one shopping trolley (2) to be decontaminated can be introduced into the decontaminating passage (3), as contents for this decontaminating passage (3), for decontamination and through which the decontaminated contents of this decontaminating passage (3) can be removed from the decontaminating passage (3) after decontamination;
- a door (6) which is displaceable between a position in which this door (6) closes off the access opening (13) in a closed state and a position in which this door (6) opens up the access opening (13) in an open state;
- a second decontaminating passage (3) which is provided with an access opening (13) through which at least one shopping trolley (2) to be decontaminated can be introduced into the decontaminating passage (3), as contents for this decontaminating passage (3), for decontamination and through which the decontaminated contents of this decontaminating passage (3) can be removed from the decontaminating passage (3) after decontamination;
- one or more decontaminating units (9) for alternately decontaminating the contents of each of the decontaminating passages (3) when the access opening (13) is in the closed state;
**characterized in that** the decontamination device (1) comprises a third decontaminating passage (4, 5) which is arranged adjacent to the first two decontaminating passages (4, 5) and which is provided with an access opening (13) through which at least one shopping trolley (2) to be decontaminated can be introduced into this decontaminating passage (4, 5), as contents for this decontaminating passage (4, 5), for decontamination and through which the decontaminated contents of this decontaminating passage (4, 5) can be removed from this decontaminating passage (4, 5) after decontamination, wherein the access openings (13) of the decontaminating passages (3, 4, 5) are arranged adjacent to each other, and wherein the one or more decontaminating units (9) are provided for alternately decontaminating the contents of each of these decontaminating passages (3) when the respective access opening (13) is in the closed state, further **characterized in that** the door (6) is displaceable across the access openings (13) in order to alternately close off the access openings (13) in a closed state and open them up again in an open state, **in that** the decontamination device (1) comprises a roof (7) which is displaceable across the decontaminating passages (3, 4, 5) in order to alternately close off each of the decontaminating passages (3, 4, 5) at the top, and **in that** the one or more decontaminating units (9) are integrated into the displaceable roof (7).

2. Decontamination device (1) according to Claim 1, **characterized in that** the displaceable roof (7) and the displaceable door (6) form part of the same displaceable roof/door unit (8).

3. Decontamination device (1) according to Claim 2, **characterized in that** the roof/door unit (8) is manufactured with a folded metal sheet.

4. Decontamination device (1) according to one of the preceding claims, **characterized in that** the displaceable door (6) is slidable across the access openings (13) in order to alternately close off these access openings (13).

5. Decontamination device (1) according to one of the preceding claims, **characterized in that** the decontamination device (1) comprises signalling means (16) in order to indicate, for each decontaminating passage (3, 4, 5), whether:
- the access opening (13) thereof is being closed, and when this access opening (13) is in the closed state, whether the contents of this decontaminating passage (3, 4, 5) are being decontaminated; or
- the access opening (13) thereof is opened in order to remove the decontaminated contents of this decontaminating passage (3, 4, 5) from the decontaminating passage (3, 4, 5) through said access opening after decontamination; or
- the access opening (13) thereof remains open in order to introduce at least one shopping trolley (2) to be decontaminated through said access opening into the decontaminating passage (3, 4, 5), as contents for this decontaminating passage (3, 4, 5), after the decontaminated contents of this decontaminating passage (3, 4, 5) have been removed from the decontaminating passage (3, 4, 5) through said access opening.

6. Decontamination device (1) according to one of the preceding claims, **characterized in that** the decontamination device (1) comprises a control unit (18) for controlling the displacement movement of the displaceable door (6) and for controlling the one or more decontaminating units (9) in order to decontaminate the contents of each of the decontaminating passages (3, 4, 5).

7. Decontamination device (1) according to Claim 5 and Claim 6, **characterized in that** the control unit (18) is provided for controlling the signalling means (16).

8. Decontamination device (1) according to Claim 6 or 7, **characterized in that** the control unit (18) is configured to control the displacement movement and the decontaminating units (9) in such a way that the following alternately takes place in each case:
- the access opening (13) of one of the decontaminating passages (3, 4, 5) is closed off, and when it is in the closed state, the contents of this decontaminating passage (3, 4, 5) are decontaminated;
- the access opening (13) of one of the decontaminating passages (3, 4, 5) is opened in order to remove the decontaminated contents of this decontaminating passage (3, 4, 5) from the decontaminating passage (3, 4, 5) through said access opening after decontamination; and
- the access opening (13) of one of the decontaminating passages (3, 4, 5) remains open in order to introduce at least one shopping trolley (2) to be decontaminated through said access opening into the decontaminating passage (3, 4, 5), as contents for this decontaminating passage (3, 4, 5), after the decontaminated contents of this decontaminating passage (3, 4, 5) have been removed from the decontaminating passage (3, 4, 5) through said access opening.
